# EUROPEAN PATENT SPECIFICATION

(11) **EP 2 755 474 B1**
(45) Date of publication and mention of the grant of the patent: **02.11.2016**
(21) Application number: 12769694.6
(22) Date of filing: 14.09.2012
(51) Int. Cl.: A01N 59/00, A01N 25/04, A61K 8/02, A61K 8/04, A61K 8/25, A61K 8/37, A61Q 19/00, C09D 5/14, C09D 5/16, A01P 1/00

(54) **ANTIMICROBIAL GELS COMPRISING BIOACTIVE GLASSES AND APPLICATIONS THEREOF**
ANTIMIKROBIELLE GELE ENTHALTEND BIOAKTIVE GLÄSER UND ANWENDUNGEN DAVON
GELS ANTIMICROBIENNES COMPRENANT DES VERRES BIOACTIFS ET LEURS APPLICATIONS

(30) Priority: 16.09.2011 US 201161535582 P; 03.10.2011 FI 20115968
(43) Date of publication of application: 23.07.2014
(73) Proprietor: Oy Granula AB LTD, 48100 Kotka (FI)
(72) Inventor: AHLNÄS, Thomas, 48130 Kotka (FI); AHLNÄS, Johannes, 00790 Helsinki (FI)
(74) Representative: Turun Patenttitoimisto Oy
(86) International application number: PCT/FI2012/050893
(87) International publication number: WO 2013/038065

(56) References cited:
- WO-A1-2006/070971
- WO-A2-2010/115041
- US-A1- 2002 086 039
- HONG XU ET AL: "Preparation and characterization of bioglass/polyvinyl alcohol composite hydrogel", BIOMEDICAL MATERIALS, INSTITUTE OF PHYSICS PUBLISHING, BRISTOL, GB, vol. 2, no. 2, 1 June 2007 (2007-06-01), pages 62-66, XP020125608, ISSN: 1748-605X, DOI: 10.1088/1748-6041/2/2/002

## Description

### FIELD OF THE INVENTION

The present invention relates to gels prepared from anhydrous suspensions of bioactive glasses in triglyceride esters by addition of water and the use of such gels as antimicrobial active.

### BACKGROUND OF THE INVENTION

Many current antimicrobial actives are difficult to handle and dispose. Specially, in households as well as in cosmetic industry molecular synthetic antimicrobial actives are biodegrading too slowly and therefore pose a problem in sewage water, for example. Also, many active ingredients lose their activity against microbes, fungi and mould due to resistant strain development over time. For example, parabens, triclosan and many other preservatives that are used today maintain their functionality for a long time and may harm for instance biological sewage treatment systems. They also tend to provoke development of resistant strains.

Bioactive glass is a known bioactive material. Unlike most other bioactive materials, it is easy to control the manufacturing properties of bioactive glass, the rate of its chemical reactions and the biological response caused by it by changing the chemical composition of the bioactive glass itself. Bioactive glass has been used in different types of implants, such as bone fillers/substitutes, bone growth promoting materials, middle ear prostheses etc. Some compositions of bioactive glasses are known to have antimicrobial effects e.g. US 6,190,643 B1 and US 6,342,207.

These glasses could thus be used in for example cosmetics, as they do not penetrate the skin, as other antimicrobial agents do. A problem in the use of said bioactive glasses is that the particles are of such dimensions that the user can feel them in her/his fingers. Bioactive glass could also be used in dentistry, but the same problem of large particles prevents this use, at least to the extent that the products would not be comfortable to use.

Typically glass particles are ground within an aqueous media, but the problem in these applications is that as glass particles dissolve in water, crystalline growth occurs, leading to larger particles than intended in the final product. As little as 10-15 % of water is sufficient for this problem to occur. Crystalline growth of glass can be measured for example with optical microscope, light scattering microscope or diffraction microscope.

A method for preparation of solid particles for UV protection is disclosed in EP 804 144. This document presents the manufacturing of metal oxide particles by dispersing them in a molten or viscous wax and by grinding the obtained mixture. The wax is left in the mixture to avoid agglomeration of the particles.

Further *in-situ* gelling using thermosetting hydrogels have been disclosed (US 5,318,780).

Xu et al disclose the preparation of a hydrogel comprising a bioactive glass (58S: 60% SiO2, 36% CaO, 4% P2O5), ethanol, polyvinyl alcohol and water (Biomedical Materials 2007, 2, 62-66).

US2002/086039 discloses gels of bioactive glasses in anhydrous media.

However, there still exists a need for providing an environmental friendly antimicrobial material for various uses.

### OBJECT AND SUMMARY OF THE INVENTION

The object of the present invention is to provide an antimicrobial gel.

Further objectives of the present invention is to provide uses of the gel.

Another object of the present invention is to provide a method for obtaining the gel of the invention.

The present invention provides a gel obtainable by contacting an anhydrous suspension comprising
a) particles of bioactive glass wherein the mean particle size of the particles is less than or equal to 20 µm; and
b) an ester which is a triglyceride with a molecular weight of less than or equal to 100000;
with water by adding from 0.06 to 30 vol/vol-% water to the suspension;
wherein the bioactive glass comprises:
SiO₂ in an amount of 40 to 70 wt-%,
CaO in an amount of 0 to 25 wt-%,
Na₂O in an amount of 0 to 30 wt-%, and
P₂O₅ in an amount of 0 to 5 wt-%.

The present invention further provides uses of the gel in cosmetic compositions, food and feed and a variety of coating composition.

The present invention also provides a method for preparing a gel comprising the steps of
a) wet-milling bioactive glass in a non-aqueous liquid to obtain an anhydrous suspension; and
b) gelling the obtained anhydrous suspension by adding water to the suspension;
wherein
i) said obtained anhydrous suspension comprises particles of bioactive glass wherein the mean particle size of the particles is less than or equal to 20 µm; and
ii) said non-aqueous liquid comprises an ester which is a triglyceride with a molecular weight or molecular weights of less than or equal to 100000;
wherein the bioactive glass comprises:
SiO₂ in an amount of 40 to 70 wt-%,
CaO in an amount of 0 to 25 wt-%,
Na₂O in an amount of 0 to 30 wt-%, and
P₂O₅ in an amount of 0 to 5 wt-%.

### BRIEF DESCRIPTION OF THE DRAWINGS

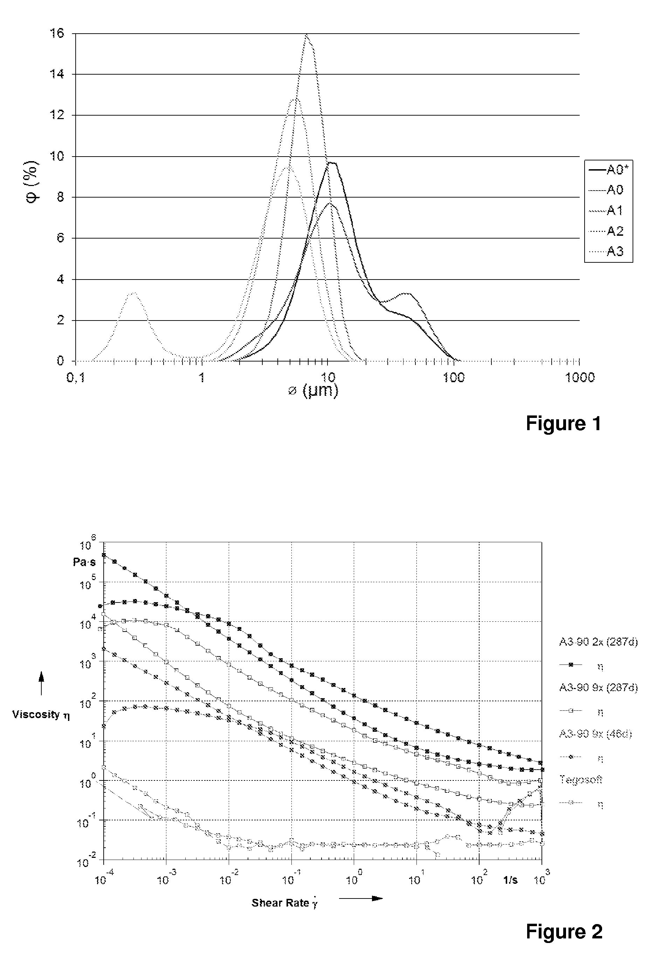

Figure 1 illustrates the particle size distribution of the dispersion A at different times of the grinding process.
Figure 2 illustrates the flow curves back and forth for a few different gels. Caprylic/capric triglyceride (trade name Tegosoft) was used as a reference.
Figure 3 illustrates how the dispersion A3H is turning into a gel. The second sample A3-90 2x illustrates the heat stability of the gel at 75 °C.
Figure 4 illustrates the complex viscosity as a function of frequency for a few gels. Again Tegosoft is as a reference.

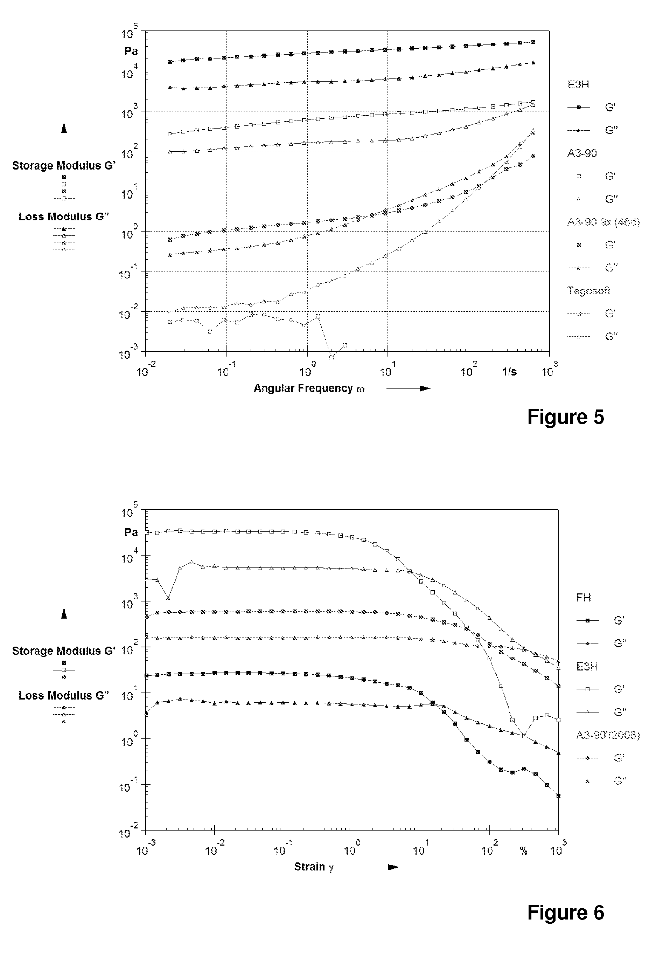

Figure 5 illustrates the modules as a function of frequency for a few gels. Again Tegosoft is as a reference.
Figure 6 illustrates the linear-viscoelastic region for a few gels. The modules are depicted as a function of strain.

### DETAILED DESCRIPTION OF THE INVENTION

The present invention relates to an antibacterial composition which can be gelled by the addition of water and which further is environmental friendly.

Bioactive glass material for the present invention can be manufactured according to prior art. In short the silicate can be made by traditional melting, flame-synthesis, sol-gel method or preferably by the cold-crust method. For the cold-crust method the oxide ingredients can be mixed and added to a kiln continuously or semi-continuously so that a crust covers the melting glass at all times.

During the glassmaking process other raw materials might also be added. For example TiO₂, ZnO and/or CeO₂ with a primary crystal size of 5-300 nm might be added to enhance the adsorption/reflexion of ultraviolet rays. Care should be taken that the temperature doesn't exceed the melting temperature of the additives.

The silicate material can be ground using (a) suitable method(s) to a desired particle size preferably of less than 20 µm. The size distributions were measured by laser diffraction.

Grounding can be done by dry-grinding or crushing with e.g. a jet-mill, a roller-mill a hammer-mill etc., but preferably by wet-milling in a colloidal mill. As media a non-aqueous liquid is chosen which comprises an ester which is a triglyceride with a molecular weight of less than or equal to 100000.

A dispersing agent and/or emulsifier/surfactants/polymers/particles or similar additives can also be included, but aren't necessary.

Optimization of size reduction can be done in order to prevent unwanted contamination and to achieve the optimal size distribution(s) for the end application.

The suspension can be gelled by adding a small amount of water to the suspension. The theoretical amount of water needed for complete hydrolysis can be calculated from the molecular formula and the mole amount of the target compound; however a smaller amount can also be used.

### Definitions

By the term "gel" is meant a semi-solid material which is composed of a solid matrix/network and a fluid media.

The term coating refers to a relatively thin layer of a composition usually associated with a substrate.

Slurries and suspensions referred to in this application are dispersions.

### Preferred embodiments of the invention

Typical anhydrous suspensions for the invention comprise
a) particles of bioactive glass wherein the mean particle size of the particles is less than or equal to 20 µm, preferably 10 µm and most preferably 5 µm; and
b) an ester which is a triglyceride with a molecular weight or molecular weights of less than or equal to 100000, preferably 30000, more preferably 10000, even more preferably 3000 and most preferably 1000.

The bioactive glass employed typically comprises:
SiO₂ in an amount of 40 to 70 wt-%, preferably 50 to 65 wt-%,,
CaO in an amount of 0 to 25 wt-%, preferably 10 to 25 wt-%,
Na₂O in an amount of 0 to 30 wt-%, preferably 20 to 30 wt-%, and
P₂O₅ in an amount of 0 to 5 wt-%, preferably 2 to 5 wt-%.

In the embodiments of the invention the anhydrous suspension for the invention comprises an ester which is a triglyceride, preferably a medium chain (6 to 12 carbons) or long chain (13 to 22 carbons) triglyceride, most preferably a caprylic/capric triglyceride. In some preferred embodiments of the invention the ester or esters are comprised in oils e.g. sunflower oil and olive oil.

In some preferred embodiments of the invention the suspension of the invention further comprises a dispersing agent, preferably a polyester of a hydroxy fatty acid, more preferably a polyester of a long chain (13 to 22 carbon chain) hydroxy fatty acid, and most preferably polyhydroxystearic acid.

In many embodiments of the invention the anhydrous suspension of the invention preferably additionally comprises multiple esters or amides, and/or one or more alcohols. Accordingly additional components of the invention can include e.g. sunflower oil, olive oil, glyceride, 1,3-propanediol, propylene glycol, butylene glycol, pentylene glycol, isopropanol and any combinations thereof. Such additional components can comprise a substantial part of the suspension, preferably from 10 to 90 vol-%, more preferably from 30 to 60 vol-% and most preferably 40 to 50 vol-%.

The gel of the invention is obtainable by contacting the anhydrous suspension of the invention with water. Typically the gel is obtained by contacting of the anhydrous suspension with water is by adding from 0.06 to 30 vol/vol-%, preferably from 0.20 to 10.0 vol/vol-%, more preferably from 0.60 to 3.0 vol/vol-% and most preferably from 2.0 to 3.0 vol/vol-% water to the suspension.

It is to be understood that anhydrous suspension can be sprayed onto a solid surface and if sufficient water is provided or available a gel of the invention is formed as a coating on the sprayed surface. This approach can be applicable in many preferred embodiments.

The gel according to the invention is typically used as an antimicrobial active.

In many embodiments of the invention the gel is used in cosmetic compositions. Cosmetic compositions can be compositions protecting against UV light. Some especially preferred such composition comprise a pigment made up of metal oxide particles having a mean primary particle diameter smaller than 0.150 µm and reducing the penetration of UV light, wherein the protector is in the form of solid particles which have a mean diameter of at minimum 10 µm and which contain 10-80 parts by weight of the said pigment reducing the penetration of UV light dispersed in 90-20 parts by weight of wax.

In many other embodiments of the invention the gel is used in food or feed.

In still other embodiments of the invention the gel is used in coating compositions. Coating compositions can be paint or coating compositions for coating cardboard or paper. Coating compositions can further be marine coatings, fire retardants and/or for wood treatment.

Embodiments of the invention include composition for preserving green forage, fresh cuttings, grain, or other organic material, typically against hazardous microorganisms. In some preferred embodiments gels of these embodiments containing an ester of unsubstituted or substituted benzoic acid, wherein said composition comprises at least ester component 1) which is an ester of the unsubstituted or substituted benzoic acid with a C₁-C₉ alcohol, or a mixture of such esters, and another ester component 2) which acts synergistically with said ester component 1) and which is an ester of an unaromatic C₁-C₂₀ carboxylic acid with a C₁-C₉ alcohol.

Some preferred embodiments comprise use in a composition comprising a compound mixture and a carrier agent, wherein the compound mixture is obtained by pulverizing wood material and/or extracting wood material so that said compound mixture contains at least two different compounds, selected from the group consisting of lignans, stilbenes, juvabiones, favonoids, betulin, betulonic acid, betulinic acid, and betuloinic acid, and ester derivatives or ether derivatives or stereoisomers of said compounds, said compound mixture containing also oligeomers of lignans or stilbenes or juvabiones or flavonoids; providing that the compound mixture contains oligomers of said lignans or stilbenes or juvabiones or flavonoids 1-31 wt-%, lignans or ester derivatives or ether derivatives or stereoisomers thereof 50.0-99.9 wt-%, at least one compound selected from the gruoup consisting of 7-hydroxymatairesinol, conidendrin, conidendric acid, alpha-conidendrin, alpha-conidendric acid, isohydroxymatairesinol, cyclolaricresinol, secoisolariciresinol, anhydrosecoisolariciresinol and stilbenes as well as their ester derivatives and stereoisimers, and a carrier agent wherein the amount of the compound mixture is in a range of 0.1-5 wt-% from the total weight of the composition, providing that the range is also such that the total cytotoxicity of said compound mixture dissolved in ethanol, measured for a Ha Cat cell culture after 24 h incubation period, is lower than the cytotoxicity of 0.02-0.1 wt-% butylated hydroxyl toluene (BHT) dissolved in ethanol for a HaCat cell culture after 24 h intubation period.

In many embodiments the gel according to the invention provides additional and/or alternative advantages than that of comprising antimicrobial activity. The gel provides a stable product, e.g. a stable emulsion.

### EXAMPLES

The following examples are given to illustrate the invention and should not be read to limit the scope of the invention as claimed in any fashion.

Bioactive glass material was manufactured according to prior art. In short the silicate can be made by traditional melting, flame-synthesis, sol-gel method or preferably by the cold-crust method. For the cold-crust method the oxide ingredients were mixed and added to a kiln continuously or semi-continuously so that a crust was covering the melting glass at all times.

During the glassmaking process other raw materials might also be added. For example TiO₂, ZnO and/or CeO₂ with a primary crystal size of 5-300 nm might be added to enhance the adsorption/reflexion of ultraviolet rays. Care should be taken that the temperature doesn't exceed the melting temperature of the additives. The silicate material was ground using (a) suitable method(s) to a desired particle size preferably of less than 20 µm. The size distributions were measured by laser diffraction.

The grounding can be done by dry-grinding or crushing with e.g. a jet-mill, a roller-mill a hammer-mill etc., but preferably by wet-milling in a colloidal mill.

As media a non-aqueous liquid is chosen which comprises an ester which is a triglyceride with a molecular weight of less than or equal to 100000.

A dispersing agent and/or emulsifier/surfactants/polymers/particles or similar additives can also be included, but aren't necessary.

Optimization of the size reduction was done in order to prevent unwanted contamination and to achieve the optimal size distribution(s) for the end application.

The suspension was gelled by adding a small amount of water to the suspension. The theoretical amount of water needed for complete hydrolysis can be calculated from the molecular formula and the mole amount of the target compound; however a smaller amount can also be used.

### Example 1

### Antibacterial gels/concentrates

Dispersions of the following compositions were made:
10 w-% silicate (A, B, C and D),
2,8 w-% dispersing agent, poly(hydroxystearic acid)
87,2 w-% Caprylic/capric triglyceride.

Where the silicate compositions were by XRF-measurements the following:

| Glass | Composition (*ω*-%) | | | |
|---|---|---|---|---|
| | SiO₂ | CaO | Na₂O | P₂O₅ |
| A | 52,5 | 19,3 | 22,8 | 3,3 |
| B | 56,0 | 15,9 | 25,1 | 3,0 |
| C | 56,5 | 15,7 | 25,5 | 2,0 |
| D | 59,8 | 11,4 | 26,1 | 2,4 |

The particle size distribution for the dispersion of silicate A was the following as a function of grinding time:

| Milling time (min) | 0* | 0 | 30 | 60 | 120 |
|---|---|---|---|---|---|
| | | Diameter (*µ*m) | | | |
| Meanvalue ± standard deviation | 18 ± 17 | 17 ± 15 | 7,2 ± 2,5 | 5,4 ± 2,2 | 3,9 ± 2,6 |
| Mode 1 | 10 | 10 | 6,7 | 5,9 | 5,1 |
| Mode 2 | 39 | - | - | - | 0,3 |
| Median | 13 | 13 | 7,4 | 5,4 | 4,1 |
| 90th percentile | - | 39 | - | - | - |
| 97th percentile | 70 | - | 13 | 11 | 9,9 |
| 99th percentile | 83 | 79 | 15 | 13 | 12 |

| | | | | | |
|---|---|---|---|---|---|
| * No ultrasonification before measurement. | | | | | |

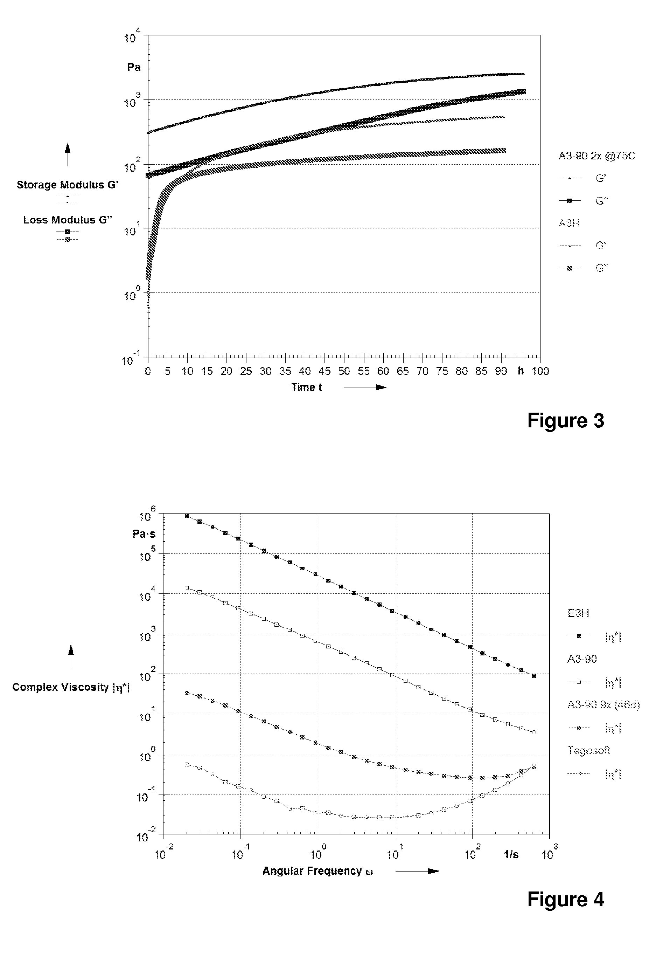

By addition of a small amount of water and thorough mixing the gelling was noticeable in a short while seen in figure 3 (A3H). The gelling time was dependent on the silicate composition and occurred immediately if the dispersing agent was omitted (dispersion E).

With these examples gels of the following strengths measured by a rheometer were acquired.

| Sample | Time (d) | Strain (%) | Shear stress (Pa) | Damping factor |
|---|---|---|---|---|
| A3-90 2x | 1 | 23,9 | 1,7 | 0,35 |
| A3-90 2x | 46 | 8,9 | 7,1 | 0,29 |
| A3-90 9x | 46 | 33,0 | 0,6 | 0,46 |
| A3-90 9x | 67 | 19,5 | 1,2 | 0,27 |
| A3-90 | | 5,1 | 28 | 0,31 |
| B3H | | 8,2 | 23 | 0,28 |
| C3H | | 6,9 | 16 | 0,31 |
| D3H | | 11,4 | 7,3 | 0,37 |
| E3H | | 0,5 | 145 | 0,18 |
| FH | | 0,6 | 0,1 | 0,26 |

Where the strain and shear stress values mark the end of the linear viscoelastic region. In the following table the crossover points (tanδ =1) for the different gels are represented.

| Sample | Time (d) | Angular frequency (rad/s) | Storage modulus (Pa) |
|---|---|---|---|
| A3-90 2x | 1 | 45 | 16 |
| A3-90 2x | 46 | 261 | 267 |
| A3-90 9x | 46 | 6 | 2 |
| A3-90 9x | 67 | 29 | 12 |
| A3-90 | | 628* | 1646 |
| E3H | | 628* | 52950 |
| B3H | | 544 | 960 |
| C3H | | 305 | 579 |
| D3H | | 150 | 180 |
| FH | | 628* | 727 |

| | | | |
|---|---|---|---|
| Crossover not yet reached at this frequency. | | | |

The sample abbreviations above can be interpreted as follows: The first letter refers to the composition used for the production of the glass as has been disclosed for A, B, C and D in the table above. These letters are only used for glass dispersion that have been both dry-ground and wet-milled employing poly(hydroxystearic acid) (PHSA) as a dispersing agent. The letters E and F refer to the same composition as A, but without PHSA; E has been both dry-ground and wet-milled whereas F has only been dry-ground. A number after a hyphen refers to the time (in minutes) wet-milled. The letter H refers to that water has been added to the glass dispersion. If *nx,* where n is an integer, is included in the abbreviation, n refers to ratio of the triglyceride to the silicate (including the dispersing agent if present).

### Example 2

### Antibacterial soap

Disclosed herein is using silicate-particles in the making of environmentally friendly antibacterial bar or preferably liquid soaps. [Garzena, Patrizia - Tadiello, Marina (2004), Soap Naturally - Ingredients, methods and recipes for natural handmade soap, (http://www.soap-naturally.com) ISBN 978-0-9756764-0-0; Thomssen, E. G., Ph. D. (1922), Soap-Making Manual, free ebook at Project Gutenberg (http://www.gutenberg.org/ebooks/34114); Mohr, Merilyn (1979), The Art of Soap Making, A Harrowsmith Contemporary Primer, Firefly Books, ISBN 978-0920656037; Dunn, Kevin M. (2010) Scientific Soapmaking: The Chemistry of Cold Process, Clavicula Press, ISBN 978.1935652090]

### Example 3

### Paper coating

Disclosed herein is the use of silicate-particle dispersions (not according to the invention). One embodiment of the invention is using gels obtained from anhydrous suspensions of silica particles as such or in combination with paper coating materials known to the art, such as those described in patent application US 7371796 or US 3784596.

Uses of this product include products that require and/or benefit of antibacterial properties, degradability and environmental soundness. The coating can be used e.g. as packaging and disposable wipes.

### Example 4

### Abrasive/ antislip material

Disclosed herein is the use of silicate-particle dispersions (not according to the invention). One embodiment of the invention is using gels obtained from anhydrous suspensions of silica particles in combination with Cerium dioxide or Betulin.

### Example 5

### Antifouling/sloughing material

Disclosed herein is the use of silicate-particle dispersions (not according to the invention). One embodiment of the invention is using gels obtained from anhydrous suspensions of silica particles in a way described in patent application US 5,990,043:
The formulations mentioned can be prepared in a manner known per se, for example by mixing the active substances with solvents and/or diluents, emulsifier and/or binder or fixative, water repellent, if desired siccatives and UV stabilizers, and, if desired, dyes and pigments, and also further processing auxiliaries.

The solvent and/or diluent is an organic-chemical solvent or solvent mixture and/or an oily or oil-like, relatively non-volatile organic-chemical solvent or solvent mixture and/or a polar organic-chemical solvent or solvent mixture and/or water and at least one emulsifier and/or wetting agent or consists thereof.

As organic-chemical solvents it is preferred to employ oil or oil-like solvents having an evaporation number of more than 35 and a flash point of more than 30° C, preferably more than 45° C. As such relatively non-volatile, water-insoluble, oily and oil-like solvents, use is made of appropriate mineral oils or their aromatic fractions or mineral oil-containing solvent mixtures, preferably white spirit, petroleum and/or alkyl benzene.

Those employed with advantage are mineral oils having a boiling range of from 170 to 220° C, white spirit having a boiling range of from 170 to 220° C, spindle oil having a boiling range of from 250 to 350° C, petroleum or aromatics with a boiling range from 160 to 280° C, turpentine oil and the like.

In a preferred embodiment, use is made of liquid aliphatic hydrocarbons having a boiling range of from 180 to 210° C or high-boiling mixtures of aromatic and aliphatic hydrocarbons having a boiling range of from 180 to 220° C. and/or spindle oil and/or monochloronaphthalene, preferably α-monochloronaphtalene. The organic, relatively non-volatile oily or oil-like solvents having an evaporation number of more than 35 and a flash point of above 30° C. preferably of above 45° C, can be replaced in part by readily or moderately volatile organic-chemical solvents, with the proviso that the solvent mixture likewise has an evaporation number of more than 35 and a flash point of more than 30° C, preferably more than 45° C, and that the insecticide/fungicide mixture is soluble or emulsifiable in this solvent mixture.

In accordance with a preferred embodiment, part of the organic-chemical solvent or solvent mixture is replaced or an aliphatic polar organic chemical solvent or solvent mixture. Preferably, aliphatic organic-chemical solvents containing hydroxyl and/or ester and/or ether groups are used, for example glycol ethers, esters or the like.

### Example 6

### UV-protecting compositions

Disclosed herein is the use of silicate-particle dispersions (not according to the invention). One embodiment of the invention is using gels obtained from anhydrous suspensions of silica particles in UV-protecting concentrates or end products such as described in patent applications:
WO 94/28867: Solid protector against UV, process for its preparation and use thereof;
US 5,603,863: Water in oil emulsions: End products containing smaller than 200 nm metallic oxide primary crystal particles;
US 5,543,135: Water in oil emulsions: Method of producing the emulsion using oil dispersions of TiO₂, ZnO and/or Fe-oxide particles less than 200 nm in primary crystal size;
US 5,516, 457 (and US 5,443,759): Oil in water emulsions: containing 0,5-30 % of smaller than 200 nm metallic oxide primary crystal particles; method of producing Oil in water emulsions using a water dispersion of less than 200 nm metallic oxide primary crystal particles;
US 5,599,529 (GB 1987 000012752): Oil dispersion of TiO₂ particles (more than 40 %) having an average size from 10 to 150 nm and an organic dispersing agent; production method;
US 5,068,056: Water dispersion (20 to 60 %) of acicular TiO₂;
US 5,366,660: Oil dispersion of ZnO particles (more than 30 %) having an average size from 5 to 150 nm and an organic dispersing agent; production method;
US 5,573,753: Method of preparing sunscreens: oil dispersion of ZnO (5-150 nm) and TiO₂ milled and then mixed with cosmetically acceptable materials and
Oil dispersions of TiO₂ and oil dispersions of ZnO EP 0 535 972.

One embodiment of a UV-protecting dispersion concentrate is
5 - 15wt-% of silicate-particles
40 - 60 wt-% oil/emollient
3 - 5 wt-% polyhydroxistearic acid
50 - 40 wt-% micronized TiO₂ for UV protection

One other embodiment of the UV-protecting end product is the use of 5-40 wt-% of the above described concentrate in an oil-in-water emulsion.

### Example 7

### Wood preserving and protecting formulations.

Disclosed herein is the use of silicate-particle dispersions (not according to the invention). One embodiment of the invention is using gels obtained from anhydrous suspensions of silica particles in combination with compositions of Patent Method treating wood US 7,812,055 and Compositions

A typical aqueous solution for wood termite protection contains said wood preservative solution 0.01-10 % by weight (for example phosphonate based HEDP) and the ammonium carboxylate of formula (1) and 1 - 45 % by weight of chelative agent containing wood preservative active ingredient, the remainder being substantially water, additives and surfactants.

A typical aqueous solution for fire protection (and termites) contains said wood preservative solution (for example phosphonate based HEDP) 5 -35 % by weight and the ammonium carboxylate of formula (1) 1-30 % by weight of chelative agent containing wood preservative active ingredient, the remainder being substantially water, additives and surfactants.

Preferably the chelating agent according to the invention is a chelating agent being able to bind iron and manganese ions and that contain phosphorus (P) in the molecular structure such as HED.

Example of a wood-protecting composition against insects, especially against termites and ants.

### Composition 7a

Composition 7a is primarily targeted as repellant against insects, especially against termites.

Composition: 30 wt-% ammonium carboxylate (43 wt-% formic acid and 57 wt-% ethanolammonium), 8.33 wt-% Cublen KT600 (60 wt-% HEDP), 0.5 wt-% PHMG 20 (20 wt-% PHMG), 1 wt-% ionic tenside, rest is water.

The composition of example 7a was well absorbed and adsorbed into wood and has excellent fixation into wood material.

Example of a composition which protects wood against fire and is also useful against insects especially against termites and ants.

### Composition 7b

Composition 7b is intended primarily for protecting wood against fire but it has also repellant properties against insects such as termites.

Composition: 10 wt- % ammonium carboxylate (43 wt-% formic acid and 57 wt-% monoethanolammonium), 49.2 wt-% Cublen KT 600 (29.5 wt-% HEDP), 20.6 wt-% ammonia water (24.5 wt-%) i.e. ammonia (100%), 3.6 wt-% ionic tenside, rest is water.

The composition of example 2 was applied onto surface of plywood board made of spruce or birch. The composition was absorbed well into wood, about 250 g/m² when applied once. Treated wood material showed excellent fire-protecting properties in a test according to standard EN5660.

The compositions 7a and 7b can be used for impregnating, painting or spraying wood as described below.

The chelating agent 1- hydroxyethylidene,1,1-diphosphonic acid (HEDP) used in compositions 1 and 2 can be replaced by other phosphor containing chelating agents such as ethylenediaminetetramethylenephosphonic acid (EDTMP) or diethylenetriaiminepentamethylenephosphonic acid (DTPMP).

### Example 8

### Lignan containing concentrates

Disclosed herein is the use of silicate-particle dispersions (not according to the invention). One embodiment of the invention is using gels obtained from anhydrous suspensions of silica particles containing extracts from wood described in patent application US 2010/0129304.

This way the antioxidant and preservation effects of both materials are joined to make a multifunctional product.

Antibacterial and antioxidant concentrate example:
1-99 wt-% GranLux AOX-G3 (containing approximately 7,5 wt-% lignans in propylene glycol), the product is manufactured by Granula Ltd;
99-1 wt-% silicate-particles dispersion (not according to the invention) or gel (for example 10 wt-% silicate-particles in caprylic/capric triglyceride) produced according to present patent application.

Use of this product in technical products, food, feed or cosmetic end formulations described in US 2010/0129304.

### Example 9

### Antimicrobial effect

Samples containing olive oil, the above mentioned silicate-particles and polyhydroxystearic acid in gel suspension were tested against various microbes, mould and fungi. Specifically, the Malazzesia furfur causing dandruff was added to the tests.

The weight of each sample was 10 g, and 1 ml of a microbe cell suspension was added to each sample. The density of each suspension was at least 5·10⁶ microbes/ml. The samples were left at room temperature (22 °C) and were tested at 2, 4, 24 and 48 hours as well as after 4, 7, 14 and 28 days.

The following microbes and strains were used.
*Staphylococcus aureus* ATCC 6538,
*Escherichia coll* ATCC 8739,
*Pseudomonas aeruginosa* ATCC 9027,
*Pseudomonas putida* ATCC 49128,
*Klebsiella pneumoniae* ATCC 10031,
*Candida albicans* ATCC 10231,
*Malassezia furfur* ATCC 96809 and
*Aspergillus niger ATCC* 16404.

When it was no more possible to observe cells able to proliferate, 1 ml of a sample was cultivated in 100 ml of Letheen-solution. After a cultivation of 7 days, the eventual microbe proliferation was tested on a culture medium.

Initially, the hydrophobic silicate particle gel showed no response, but after 4 hours it had a good effect against all tested species. Also, subsequent cultivation of the test media showed that the formulation was not only bacteriostatic but also bactericidal.

The gel showed good heat stability and stability in the cold. Further the gel was not destroyed by dilution with more emollient or oil i.e. a gel concentrate can be manufactured and used to achieve an end formulation.

### Example 10

### Further components

A dispersion comprising the E glass (about 10 vol-%) and caprylic/capric triglyseride was mixed with further components selected from the group comprising sunflower oil, olive oil, glyceride, 1,3-propanediol, propylene glycol, butylene glycol, pentylene glycol and isopropanol to provide a gel according to the invention for various applications. The mixtures were made in an about 1 to 1 volume ratio. All mixtures resulted in gels albeit some of them only after substantial aging.

### Example 11

### Cosmetic end product

A day cream was prepared using the receipt:
32 wt-% E glass dispersion
31 wt-% caprylic/capric triglyceride (Tegosoft CT)
9 wt-% titanium dioxide (Sachtleben-Pigments M161)
10 wt-% water
18 wt-% poly methyl methacrylate (Micropearl M100)
No emulsifier was employed.

The end product resulted in a very nice skin feel.

## Claims

1. A gel obtainable by contacting an anhydrous suspension comprising
a) particles of bioactive glass wherein the mean particle size of the particles is less than or equal to 20 µm; and
b) an ester which is a triglyceride with a molecular weight of less than or equal to 100000;
with water by adding from 0.06 to 30 vol/vol-% water to the suspension; wherein the bioactive glass comprises:
SiO₂ in an amount of 40 to 70 wt-%,
CaO in an amount of 0 to 25 wt-%,
Na₂O in an amount of 0 to 30 wt-%, and
P₂O₅ in an amount of 0 to 5 wt-%.

2. The gel of claim 1 **characterized in that** the anhydrous suspension comprises a medium chain, with 6 to 12 carbons, or long chain, with 13 to 22 carbons, triglyceride.

3. The gel of claim 2 **characterized in that** the anhydrous suspension comprises a caprylic/capric triglyceride.

4. The gel of any of preceding claims **characterized in that** the anhydrous suspension further comprises a dispersing agent.

5. The gel of any of preceding claims **characterized in that** the anhydrous suspension further comprises multiple esters or amides, and/or one or more alcohols.

6. Use of the gel according to any of preceding claims as an antimicrobial active.

7. The use according to claim 6 **characterized in that** the gel is used in cosmetic compositions.

8. The use according to claim 6 **characterized in that** the gel is used in food or feed.

9. The use according to claim 6 **characterized in that** the gel is used in coating compositions.

10. The use according to claim 9 **characterized in that** the coating compositions are paint or coating compositions for coating cardboard or paper.

11. The use according to claim 9 **characterized in that** the coating compositions are marine coatings.

12. The use according to claim 9 **characterized in that** the coating compositions are fire retardants.

13. The use according to claim 9 **characterized in that** the coating compositions are for wood treatment.

14. A method for preparing a gel comprising the steps of
a) wet-milling bioactive glass in a non-aqueous liquid to obtain an anhydrous suspension; and
b) gelling the obtained anhydrous suspension by adding water to the suspension;
wherein
i) said obtained anhydrous suspension comprises particles of bioactive glass wherein the mean particle size of the particles is less than or equal to 20 µm; and
ii) said non-aqueous liquid comprises an ester which is a triglyceride with a molecular weight or molecular weights of less than or equal to 100000;
wherein the bioactive glass comprises:
SiO₂ in an amount of 40 to 70 wt-%,
CaO in an amount of 0 to 25 wt-%,
Na₂O in an amount of 0 to 30 wt-%, and
P₂O₅ in an amount of 0 to 5 wt-%.

15. The method according to claim 14 **characterized in that** the gelling the obtained suspension is carried out by adding from 0.06 to 30 vol/vol-% water to the suspension.

16. The method according any of claim 14 or 15 **characterized in that** it said non-aqueous liquid comprising a medium chain, with 6 to 12 carbons, or long chain, with 13 to 22 carbons, triglyceride.

17. The method according to claim 16 **characterized in that** said non-aqueous liquid comprises a caprylic/capric triglyceride.

18. The method according any of claims 14 to 16 **characterized in that** a dispersing agent, emulsifier, surfactant and/or polymer is comprised in the non-aqueous liquid wherein the bioactive glass is ground.

19. The method according any of claims 14 to 18 **characterized in that** wet-milling is carried out with a colloidal mill.

## Patentansprüche

1. Gel, erhältlich durch Inkontaktbringen einer wasserfreien Suspension, umfassend
a) Partikel aus bioaktivem Glas, wobei die mittlere Partikelgröße der Partikel kleiner als oder gleich 20 µm ist, und
b) einen Ester, welcher ein Triglycerid mit einem Molekulargewicht von kleiner als oder gleich 100.000 ist,
mit Wasser durch Hinzugeben von 0,06 bis 30 Vol/Vol-% Wasser zur Suspension,
wobei das bioaktive Glas folgendes umfasst:
SiO₂ in einer Menge von 40 bis 70 Gew.-%,
CaO in einer Menge von 0 bis 25 Gew.-%,
Na₂O in einer Menge von 0 bis 30 Gew.-% und
P₂O₅ in einer Menge von 0 bis 5 Gew.-%.

2. Gel gemäß Anspruch 1, **dadurch gekennzeichnet, dass** die wasserfreie Suspension ein Triglycerid mit einer mittleren Kette mit 6 bis 12 Kohlenstoffen oder mit einer langen Kette mit 13 bis 22 Kohlenstoffen umfasst.

3. Gel gemäß Anspruch 2, **dadurch gekennzeichnet, dass** die wasserfreie Suspension ein Capryl-/Caprin-Triglycerid umfasst.

4. Gel gemäß einem der vorangehenden Ansprüche, **dadurch gekennzeichnet, dass** die wasserfreie Suspension ferner ein Dispergiermittel umfasst.

5. Gel gemäß einem der vorangehenden Ansprüche, **dadurch gekennzeichnet, dass** die wasserfreie Suspension ferner mehrfache Ester oder Amide und/oder ein oder mehr Alkohole umfasst.

6. Verwendung des Gels gemäß einem der vorangehenden Ansprüche als antimikrobieller Wirkstoff.

7. Verwendung gemäß Anspruch 6, dadurch **gekennzeich**n e t , dass das Gel in kosmetischen Zusammensetzungen verwendet wird.

8. Verwendung gemäß Anspruch 6, dadurch **gekennzeich**n e t , dass das Gel in Nahrungs- oder Futtermitteln verwendet wird.

9. Verwendung gemäß Anspruch 6, dadurch **gekennzeich**n e t , dass das Gel in Beschichtungszusammensetzungen verwendet wird.

10. Verwendung gemäß Anspruch 9, dadurch **gekennzeich**n e t , dass die Beschichtungszusammensetzungen Farb- oder Beschichtungszusammensetzungen zur Beschichtung von Pappe oder Papier sind.

11. Verwendung gemäß Anspruch 9, dadurch **gekennzeich**n e t , dass die Beschichtungszusammensetzungen marine Beschichtungen sind.

12. Verwendung gemäß Anspruch 9, dadurch **gekennzeich**n e t , dass die Beschichtungszusammensetzungen Flammschutzmittel sind.

13. Verwendung gemäß Anspruch 9, dadurch **gekennzeich**n e t , dass die Beschichtungszusammensetzungen zur Holzbehandlung beabsichtigt sind.

14. Verfahren zur Herstellung eines Gels, umfassend die folgenden Schritte:
a) Nassvermahlen bioaktiven Glases in einer nichtwässrigen Flüssigkeit, um eine wasserfreie Suspension zu erhalten und
b) Gelieren der erhaltenen wasserfreien Suspension durch Zufügen von Wasser zu der Suspension,
wobei
i) die erhaltene wasserfreie Suspension Partikel aus bioaktivem Glas umfasst, wobei die mittlere Partikelgröße der Partikel kleiner als oder gleich 20 µm ist, und
ii) die nichtwässrige Flüssigkeit einen Ester umfasst, welcher ein Triglycerid mit einem Molekulargewicht oder Molekulargewichten kleiner als oder gleich 100.000 ist,
wobei das bioaktive Glas Folgendes umfasst:
SiO₂ in einer Menge von 40 bis 70 Gew.-%,
CaO in einer Menge von 0 bis 25 Gew.-%,
Na₂O in einer Menge von 0 bis 30 Gew.-% und
P₂O₅ in einer Menge von 0 bis 5 Gew.-%.

15. Verfahren gemäß Anspruch 14, dadurch **gekennzeich**n e t , dass das Gelieren der erhaltenen Suspension durch Hinzufügen von 0,06 bis 30 Vol/Vol-% Wasser zur Suspension durchgeführt wird.

16. Verfahren gemäß einem der Ansprüche 14 oder 15, **dadurch gekennzeichnet, dass** die nichtwässrige Flüssigkeit ein Triglycerid mit einer mittleren Kette mit 6 bis 12 Kohlenstoffen oder mit einer langen Kette mit 13 bis 22 Kohlenstoffen umfasst.

17. Verfahren gemäß Anspruch 16, dadurch **gekennzeich**n e t , dass die nichtwässrige Flüssigkeit ein Capryl-/ Caprin-Triglycerid umfasst.

18. Verfahren gemäß einem der Ansprüche 14 bis 16, **dadurch gekennzeichnet, dass** ein Dispersionsmittel, Emulgator, Surfactant und/oder Polymer in der nichtwässrigen Flüssigkeit enthalten ist, in welcher das bioaktive Glas vermahlen wird.

19. Verfahren gemäß einem der Ansprüche 14 bis 18, **dadurch gekennzeichnet, dass** die Nassvermahlung mit einer Kolloidmühle durchgeführt wird.

## Revendications

1. Gel pouvant être obtenu par mise en contact d'une suspension anhydre comprenant
a) des particules de verre bioactif, dans lesquelles la taille de particule moyenne des particules est inférieure ou égale à 20 µm ; et
b) un ester qui est un triglycéride d'un poids moléculaire inférieur ou égal à 100 000 ;
avec de l'eau par ajout de 0,06 à 30 % vol/vol d'eau à la suspension ;
dans lequel le verre bioactif comprend :
du SiO₂ dans une quantité de 40 à 70 % en poids,
du CaO dans une quantité de 0 à 25 % en poids,
du Na₂O dans une quantité de 0 à 30 % en poids, et
du P₂O₅ dans une quantité de 0 à 5 % en poids.

2. Gel selon la revendication 1, **caractérisé en ce que** la suspension anhydre comprend un triglycéride à chaîne moyenne de 6 à 12 carbones, ou à chaîne longue de 13 à 22 carbones.

3. Gel selon la revendication 2, **caractérisé en ce que** la suspension anhydre comprend un triglycéride caprylique/caprique.

4. Gel selon l'une quelconque des revendications précédentes, **caractérisé en ce que** la suspension anhydre comprend en outre un agent dispersant.

5. Gel selon l'une quelconque des revendications précédentes, **caractérisé en ce que** la suspension anhydre comprend en outre de multiples esters ou amides, et/ou un ou plusieurs alcools.

6. Utilisation du gel selon l'une quelconque des revendications précédentes, en tant qu'actif antimicrobien.

7. Utilisation selon la revendication 6, **caractérisée en ce que** le gel est utilisé dans des compositions cosmétiques.

8. Utilisation selon la revendication 6, **caractérisée en ce que** le gel est utilisé dans un aliment ou une nourriture.

9. Utilisation selon la revendication 6, **caractérisée en ce que** le gel est utilisé dans des compositions de revêtement.

10. Utilisation selon la revendication 9, **caractérisée en ce que** les compositions de revêtement sont des compositions de peinture ou de revêtement pour recouvrir du carton ou du papier.

11. Utilisation selon la revendication 9, **caractérisée en ce que** les compositions de revêtement sont des revêtements marins.

12. Utilisation selon la revendication 9, **caractérisée en ce que** les compositions de revêtement sont des produits ignifuges.

13. Utilisation selon la revendication 9, **caractérisée en ce que** les compositions de revêtement sont destinées à un traitement du bois.

14. Procédé de préparation d'un gel comprenant les étapes de
a) broyage humide du verre bioactif dans un liquide non aqueux pour obtenir une suspension anhydre ; et
b) gélification de la suspension anhydre obtenue par ajout d'eau à la suspension ;
dans lequel
i) ladite suspension anhydre obtenue comprend des particules de verre bioactif, dans lesquelles la taille de particule moyenne des particules étant inférieure ou égale à 20 µm ; et
ii) ledit liquide non aqueux comprend un ester qui est un triglycéride avec un poids moléculaire ou des poids moléculaires inférieurs ou égaux à 100 000 ;
dans lequel le verre bioactif comprend :
du SiO₂ dans une quantité de 40 à 70 % en poids,
du CaO dans une quantité de 0 à 25 % en poids,
du Na₂O dans une quantité de 0 à 30 % en poids, et
du P₂O₅ dans une quantité de 0 à 5 % en poids.

15. Procédé selon la revendication 14, **caractérisé en ce que** la gélification de la suspension obtenue est réalisée par ajout de 0,06 à 30 % vol/vol d'eau à la suspension.

16. Procédé selon l'une quelconque des revendications 14 ou 15, **caractérisé en ce que** ledit liquide non aqueux comprend un triglycéride à chaîne moyenne de 6 à 12 carbones, ou à chaîne longue de 13 à 22 carbones.

17. Procédé selon la revendication 16, **caractérisé en ce que** ledit liquide non aqueux comprend un triglycéride caprylique/caprique.

18. Procédé selon l'une quelconque des revendications 14 à 16, **caractérisé en ce qu'**un agent dispersant, un émulsifiant, un tensioactif et/ou un polymère sont compris dans le liquide non aqueux dans lequel le verre bioactif est broyé.

19. Procédé selon l'une quelconque des revendications 14 à 18, **caractérisé en ce que** le broyage humide est réalisé avec un moulin colloïdal.
